(19)

Europäisches
Patentamt
European
Patent Office
Office européen
des brevets

(11) **EP 2 386 297 A1**

(12) **EUROPEAN PATENT APPLICATION**

(43) Date of publication:
**16.11.2011 Bulletin 2011/46**

(51) Int Cl.:
**A61K 9/50** *(2006.01)* **A61K 9/20** *(2006.01)*
**A61K 47/38** *(2006.01)* **A61K 47/36** *(2006.01)*
**A61K 9/28** *(2006.01)* **A61K 9/48** *(2006.01)*

(21) Application number: **10305452.4**

(22) Date of filing: **28.04.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicant: **Roquette Frères**
**62136 Lestrem (FR)**

(72) Inventors:
• **Siepmann, Juergen**
**59133 Phalempin (FR)**
• **Karrout, Youness**
**59000 Lille (FR)**
• **Guerin-Deremaux, Laëtitia**
**59850 Nieppe (FR)**
• **Klaeyle, Jérôme**
**59000 Lille (FR)**
• **Wils, Daniel**
**59190 Morbecque (FR)**

(74) Representative: **Boulinguiez, Didier et al**
**Cabinet Plasseraud**
**52, rue de la Victoire**
**75440 Paris Cedex 09 (FR)**

(54) **Indigestible polymer: starch acetate -based film coatings for colon targeting**

(57) The present invention provides a colon targeted delivery dosage form for controlled release of an active ingredient, comprising an active ingredient coated in a polymeric mixture of:
o a water insoluble polymer composition containing at least a starch acetate, and

o an indigestible polysaccharide composition

The present invention also relates to the use and method for making the same.

**EP 2 386 297 A1**

**Description**

**FIELD OF THE INVENTION**

The present invention relates to a colon targeting coating containing starch acetate for a dosage form for the controlled delivery of active ingredient(s). The present invention also relates to the use and method for making the same.

**BACKGROUND OF THE INVENTION**

In the treatment of diseases or ailments of the colon or rectum including the treatment of inflammatory bowel diseases, such as Crohn's Disease (CD) and Ulcerative Colitis (UC), administration of the pharmacologically active agent to the affected site may be required. Orally administrable pharmaceutical compositions however have frequently been found ineffective in this respect as a result of the absorption of the pharmacologically active agent in the digestive tract before the colon or rectum is reached.

If a locally acting drug is orally administered using a conventional pharmaceutical dosage form, the latter rapidly dissolves in the contents of the stomach, the drug is released and likely to be absorbed into the blood stream. This leads to elevated systemic drug concentrations and, thus, an increased risk of undesired side effects and at the same time to low drug concentrations at the site of action in the colon, resulting in poor therapeutic efficiency. These restrictions can be overcome if drug release is suppressed in the stomach and small intestine and time-controlled in the colon. This type of site-specific drug delivery to the colon might also offer an interesting opportunity for protein and peptide drugs to get absorbed into the systemic circulation upon oral administration.

To allow for colon targeting, the drug can for instance be embedded within a polymeric matrix former, or drug-loaded tablets or pellets (such as spherical beads, approximately 0.5-1 mm in diameter) can be coated with a polymeric film. In the upper gastro intestinal tract (GIT), the permeability of the polymeric networks for the drug should be low, whereas the macromolecular barriers must become permeable once the colon is reached. This increase in drug permeability of the polymeric networks at the site of action might be induced by: (i) a change in the pH of the contents of the GIT, (ii) a change in the quality and/or quantity of enzymes along the GIT, or (iii) significant structural changes within the dosage form occurring after a pre-determined lag-time (e.g. crack formation in poorly permeable film coatings providing pulsatile drug release patterns). Alternatively, drug release might already start in the stomach and continue throughout the GIT, at a rate that is sufficiently low to assure that drug is still inside the dosage form once the colon is reached.

**SUMMARY OF THE INVENTION**

An object of the present invention is to provide a delivery dosage form to control the rate and extent of delivery of an active ingredient, for example, without limitation, an active pharmaceutical ingredient, biological, chemical, nutraceutical, agricultural or nutritional active ingredients.

Another object of the present invention is to provide new polymeric film coatings that allow for site-specific drug targeting to the colon and that may be used for patients suffering from inflammatory bowel diseases as well as for patients with a healthy colon.

A further object of the present invention is to provide new polymeric film coatings having a sufficient mechanical stability to withstand the shear stress they are exposed to in the upper GIT (due to the gastro intestinal motility) and to withstand the potentially significant hydrostatic pressure developed within the dosage forms due to water penetration into the systems upon contact with aqueous media. Indeed, with known polymer coatings, the problem of accidental crack formation can result in premature drug release through water-filled channels.

A further object of the present invention is to provide new polymeric film coatings adjustable to the specific needs of a particular type of drug treatment e.g, osmotic activity of the drug and administered dose.

New colon targeting coating compositions has recently been described. Those coating compositions mainly contain ethyl cellulose as water insoluble filmogen polymer. Ethyl cellulose is a well-known coating polymer having good qualities for coating. Ethyl cellulose is usually used in the form of an aqueous suspension or powder. The new polymeric film coating contains according to the invention, a starch derivative preferably starch acetate in partial or total replacement of usual water insoluble coating polymers such as ethyl cellulose. In the colon targeting coating according to the invention, starch acetate is used in combination with at least an indigestible polysaccharide or preferably at least an indigestible non-starch polysaccharide.

The present invention provides a colon targeted delivery dosage form for controlled release of an active ingredient, comprising an active ingredient coated with a polymeric mixture of:

  o a water insoluble polymer composition containing at least a starch acetate, and
  o an indigestible polysaccharide composition.

Typically, the starch acetate has a degree of substitution of 0,01 to 3 preferably 1.6 to 3, notably 1.7 to 2.9, more preferably 2 to 2.8, even more preferably 2.3 to 2.8, very preferably 2.5 to 2.75.

According to a first variant of the invention the starch acetate has an amylose content of less than 55%, preferably between 15 and 45%, more preferably of between 20 and 44%, and most preferably still of between 24 and 40%, this percentage being expressed in dry weight with respect to the dry weight of starch present in said composition.

Typically, the starch acetate has a molecular weight of $10^3$ and $10^8$ g/mol, preferably $5.10^3$ and $10^7$g/mol, more preferably $10^4$ and $10^6$g/mol.

According to a second variant of the water insoluble polymer composition further contains another water insoluble polymer or a plasticizer. Preferably according to the second variant of the invention, said acetylated starch has an amylose content of less than 90% more preferably between 15 and 85%, in particular of between 20 and 80%, and notably of between 24 and 70%, most preferably 30 to 60%, very preferably still 35 to 55%, this percentage being expressed in dry weight with respect to the dry weight of starch present in said composition.

Preferentially the colon targeted delivery dosage form is an oral formulation and has a gastric resistance. In a preferred embodiment, the colon targeted delivery dosage form is in a solid form. According to the invention, the polymeric mixture is an intimate mix of the starch acetate and at least an indigestible polysaccharide contained in the indigestible polysaccharide composition.

In an embodiment of the present invention, the polymeric mixture of the colon targeted delivery dosage form is a coating mixture, the colon targeted delivery dosage form comprising a core, the active ingredient being dispersed or dissolved in the core and/or in the coating mixture.

In a further embodiment of the present invention, the indigestible polysaccharide composition:water insoluble polymer composition ratio in weight in the controlled release delivery dosage form is between 1:2 and 1:8, preferentially 1:3 to 1:6, and more preferentially 1:4 to 1:5.

Typically, the starch acetate is a powder having elementary particles size observed by Scanning electron microscopy, between 10μm to 0.05μm, preferably 5 to 3μm more preferably 1 to 0.1μm.

In a further embodiment of the invention, the starch acetate is a barley, oats, wheat, potato, legume and corn starch.

Typically, starches from barley, pea and oats has a relatively low amylose content, which is about 20-45% (w/w), more exactly 22-40 (w/w), most specifically about 25-35% (w/w).

The starch acetate according to the invention may be prepared from modified starches such as starches modified by fluidification, esterification or etherification such as for example by hydroxypropylation, cross-linking, cationization, anionization, succinylation, silylation ou telomerization or by oxidization phosphatation and the like.

The starch acetate according to the invention may also be further modified by esterification or etherification such as for example by hydroxypropylation, cross-linking, cationization, anionization, succinylation, silylation ou telomerization or by oxidization phosphatation and the like.

The water insoluble polymer composition preferably contains a mix of acetylated starches.

According to the second alternative of the invention, the water insoluble polymer composition contains a starch acetate and another water insoluble polymer, typically a non-starch-acetate water insoluble polymer. Preferably, the water insoluble polymer is selected from the group consisting of ethyl cellulose, cellulose derivatives, acrylic and/or methacrylic ester polymers, polymers or copolymers of acrylate or methacrylate polyvinyl esters, starch derivatives, polyvinyl acetates, polyacrylic acid esters, butadiene styrene copolymers methacrylate ester copolymers, cellulose acetate phtalate, polyvinyl acetate phtalate, shellac, methacrylic acid copolymers, cellulose acetate trimellitate, hydroxypropyl methylcellulose phtalate, zein.

In a further embodiment of the present invention, the ratio in weight between the starch acetate and the other water insoluble polymers is between 1:2 and 1:8, preferentially 1:3 to 1:6, and more preferentially 1:4 to 1:5.

In a further embodiment of the invention, the indigestible polysaccharide composition comprises at least one indigestible polysaccharide selected from the group consisting of starch, xylooligosaccharides, inulin, oligofructoses, fructo-oligosacharides (FOS), lactulose, galactomannan and suitable hydrolysates thereof, indigestible polydextrose, indigestible dextrin and partial hydrolysates thereof, trans-galacto-oligosaccharides (GOS), xylo-oligosaccharides (XOS), acemannan, lentinan or beta-glucan and partial hydrolysates thereof, polysaccharides-K (PSK), and indigestible maltodextrin and partial hydrolysates thereof, preferably an indigestible dextrin or an indigestible maltodextrin.

According to the invention, the indigestible maltodextrin or indigestible dextrin has between 15 and 50%, preferably between 20 and 40%, more preferably between 25 and 35%, of 1->6 glucoside linkages, a reducing sugar content of less than 20%, preferably between 2 and 20%, more preferably between 2.5 and 15%, more preferably between 3.5 and 10%, a polymolecularity index of less than 5, preferably between 1 and 4%, more preferably between 1.5 and 3%, and a number-average molecular mass Mn at most equal to 4500 g/mol, more preferably between 500 and 3000 g/mol, more preferably between 700 and 2800 g/mol, more preferably between 1000 and 2600 g/mol.

According to a variant, all or some of the said indigestible maltodextrins are hydrogenated.

According to a further variant of the invention said indigestible polysaccharide is starch selected from legume or cereal starch

Typically, the legume starch is selected from the group consisting of pea, bean, broad bean and horse bean starch.

According to another advantageous alternative form, the legume is a plant, for example a variety of pea or of horse bean, giving seeds comprising at least 25%, preferably at least 40%, by weight of starch (dry/dry). Preferably, the legume starch is a granular legume starch.

Advantageously, the legume is pea. Pea starch granules have two particularities. The first one is large granule diameter, larger than for example corn starch granules, improving the granule's surface area and thus contacts with water and micro flora enzymes in the colon. In addition, pea starch granules have a high swollen ability improving their surface area thus granules granules digestibility and consequently the active ingredient release in the colon.

According to another advantageous alternative the legume starch is a native legume starch.

Advantageously, this starch content of the indigestible polysaccharide composition is greater than 90% (dry/dry). It can in particular be greater than 95%, preferentially greater than 98%.

According to the invention, the starch of the indigestible polysaccharide composition is a modified starch preferably a stabilized starch. Indeed, according to a preferred embodiment of the invention, the chemical treatments, which are particularly well suited to the preparation of a film-forming composition, are the "stabilizing" treatments. Common stabilization modifications may be accomplished by esterifying or etherifying some of the hydroxyl groups along the starch chain. Preferentially, said modified starch is hydroxypropylated and/or acetylated; it being possible for these treatments optionally to be supplemented by a fluidification that is a chemical or enzymatic hydrolysis treatment. Preferably, said modified starch of the indigestible polysaccharide composition is fluidification-treated, for example by acid treatment. The starch composition according to the invention thus advantageously comprises at least one stabilized starch and preferably a hydroxypropylated starch exhibiting a degree of substitution (DS) of at most 0.2. The term "DS" is understood to mean, in the present invention, the mean number of hydroxypropyl groups per 10 anhydroglucose units. This mean number is determined by the standard analytical methods well known to a person skilled in the art.

According to a variant, the core has a coating level of 5% to 30%, preferably of 10% to 20%, and still more preferably of 13% to 17%.

In a further embodiment, the polymeric mixture comprises a plasticizer. Preferably the plasticizer content is between 15% to 50% w/w referred to the water insoluble polymer composition content, preferably 20 to 45% w/w, more preferably 22 to 40% w/w, even more preferably 25 to 35% w/w, very preferably 27 to 32% w/w, most preferably 28 to 30% w/w.

The plasticizer may be chosen in particular among the esters and ether of diols, triols and polyols like glycerol, polyglycerols, isosorbide, sorbitans, sorbitol, mannitol, and hydrogenated glucose syrups, the esters of organic acids, urea or any mixtures of these products.

The plasticizer may be particularly selected from methylic or ethylic esters, esters of fatty organics acids or esters of inorganic acids such as lactic, citric, succinic, adipic, sebacic, phthalic, glutaric or phosphoric acid or acetic acid or fatty esters of mono alcohols, diols, triols or polyols such as ethanol, diethylene glycol, glycerol or sorbitol. For example, we may specifically mention the glycerol diacetate (diacetin), glycerol triacetate (triacetin), triethyl citrate on the diacetate isosorbide dioctanoate isosorbide, mononitrate dioleate, dilaurate isosorbide esters of dicarboxylic acids or dibasic esters (Dibasic esters "or" DBE ") and any mixtures of these products. The plasticizer may also be epoxidized vegetable oil, a glycol derivative as a polyester or ethylene glycol.

The plasticizer may also be chosen from the above products coupled together by coupling agents such as epichlorohydrin or an isocyanate.

In another embodiment, the plasticizer is characterized by its solubility parameter (the so-called HILDEBRAND parameter), which basically refers to the existing force of attraction between molecules (such as between a polymer and its plasticizer), and particularly the variation of cohesive energy density of the plasticizer, i.e. the energy needed for its vaporization. The units of solubility parameter are expressed at 25°C in $(J.cm^{-3})^{0.5}$ or in $(MPa)^{1/2}$ (where $1 (J.cm^{-3}) 0.5 = 1 (MPa)^{1/2}$).

The plasticizer used in the invention may present a solubility parameter between 15 and 28 $(J.cm^{-3})^{0.5}$, preferably between 17 and 25 $(J.cm^{-3})^{0.5}$, more preferably between 18 and 22 $(J.cm^{-3})^{0.5}$. It may be, for example, glycerol triacetate (triacetin) presenting an HILDEBAND parameter, calculated from the latent heat of vaporization (85.74kJ/mol) or its boiling temperature (259°C), of 21 $(J.cm^{-3})^{0.5}$.

In another embodiment, the plasticizer can advantageously present a molecular weight smaller than 1500 g/mol, and in particular below 500 g/mol. The plasticizer preferably has a molecular weight greater than 18 g/mol. Ideally, the plasticizer presents a molecular weight between 150 and 450 g/mol.

The plasticizer may present simultaneously, a molecular weight between 150 and 450 g/mol, and a HILDEBRAND parameter between 18 and 22 $(J.cm^{-3})^{0.5}$ as it is particularly the case with triacetin (molar mass of 218 g/mol and HILDEBRAND parameter of 21 $(J.cm^{-3})^{0.5}$).

In a preferred embodiment, the colon targeted delivery dosage form is a multiparticulate dosage form.

The present invention also provides a method for preparing a controlled release delivery dosage form preferably a colon targeted delivery dosage form for controlled release of an active ingredient in the colon of patients having a colonic microflora imbalance or in the colon of healthy subjects, said method comprising:

o forming a polymeric mixture of:

- at least a water insoluble polymer composition containing a starch acetate, said starch acetate having preferably an amylose content of less than 55% more preferably between 15 and 45%, most preferably of between 20 and 44%, and very preferably still of between 24 and 40% this percentage being expressed in dry weight with respect to the dry weight of starch present in said composition and
- an indigestible polysaccharide composition

o coating said active ingredient in the polymeric mixture.

In a further embodiment, the step of coating the active ingredient is a coating step of a core, the active ingredient being dispersed or dissolved in the core and/or the step of coating the active ingredient is a step of dispersing or dissolving the active ingredient in the polymeric mixture.

The conditions in the gastro intestinal tract of patients suffering from inflammatory bowel diseases (e.g. Crohn's Diseases and Ulcerative Colitis) can significantly differ from those in a healthy subject. The intra- and inter-individual variability can be substantial with respect to the pH of the GIT contents, types and concentrations of enzyme-secreting bacteria as well as to the transit times within the various GIT segments. For instance, considerable amounts of bifidobacteria are generally present in the colon of healthy subjects and are able to degrade complex polysaccharides due to multiple extracellular glycosidases. However, in the disease state their concentration can be significantly reduced. For example, it was shown that the fecal glycosidase activity (especially that of β-D-galactosidase) is decreased in patients suffering from Crohn's Disease and that the metabolic activity of the colonic flora is strongly disturbed in the active disease state. Thus, the impact of the pathophysiology can be crucial and can lead to the failure of the pharmaco-treatment.

To avoid treatment failures for patients suffering from inflammatory bowel diseases, the site-specific drug delivery system must be adapted to the conditions given in the patient's colon. For instance, polymeric film coatings might be used that are degraded by enzymes, which are present in the feces of Crohn's Disease and Ulcerative Colitis patients in sufficient amounts. However, yet it is unclear which type(s) of polymers fulfills these pre-requisites.

## BRIEF DESCRIPTION OF THE DRAWINGS

Fig. 1: Dry mass of thin films consisting of different types of polymer blends (MS6 HP-PG : ethylcellulose;ratios 1: 4 and 1:3; MS6 A-PG : ethylcellulose ratios 1:4 and 1:3; ethylcellulose; AS) upon exposure to: (a) 0.1 M HCl, and (b) phosphate buffer pH 6.8.

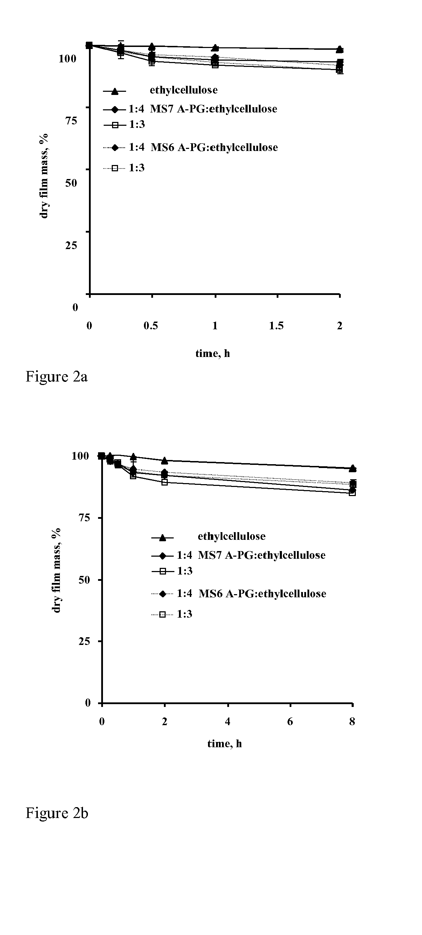

Fig. 2: Dry mass of thin films consisting of different types of polymer blends (MS7 A-PG : ethylcellulose;ratios 1:4 and 1:3; MS6 A-PG : ethylcellulose ratios 1:4 and 1:3; ethylcellulose) upon exposure to: (a) 0.1 M HCl, and (b) phosphate buffer pH 6.8.

Fig. 3: Dry mass loss kinetics of thin MS 6 HP-PG:AS films upon exposure to: (a) 0.1 M HCl, and (b) phosphate buffer pH 6.8. The polymer:polymer blend ratio (w:w) is indicated in the diagram. Films consisting only of plasticized ethylcellulose and blend of MS6 HP-PG : ethylcellulose are shown for reasons of comparison.

Fig. 4: Water content of thin MS6 HP-PG:AS films (1:3) upon exposure to culture medium inoculated with feces of ulcerative colitis (UC) patients. Films consisting only of plasticized blend of MS6 HP-PG : ethylcellulose are shown for reasons of comparison.

Fig. 5: Dry mass of thin MS6 HP-PG:AS films (1:3) upon exposure to culture medium inoculated with feces of ulcerative colitis (UC) patients. Films consisting only of plasticized blend of MS6 HP-PG : ethylcellulose are shown for reasons of comparison.

Fig 6: Water uptake kinetics of thin (AS/ethylcellulose: 1:1):BMD 4:1 films plasticized with 25 or 30% of TEC (the percentages are indicated in the diagram and refer to the ethylcellulose/AS mass) under conditions simulating the transit through the upper gastro intestinal tract: 2 h exposure to 0.1 M HCl. Films consisting only of ethylcellulose: BMD 4:1 plasticized with 25 or 30% of TEC are shown for reasons of comparison.

Fig 7: Dry mass loss kinetics of thin (AS/ethylcellulose: 1:1):BMD 4:1 films plasticized with 25% and 30 of TEC (the percentages are indicated in the diagram and refer to the ethylcellulose/AS mass) under conditions simulating the transit through the upper gastro intestinal tract: 2 h exposure to 0.1 M HCl. Films consisting only of ethylcellulose:

BMD 4:1 plasticized with 25 or 30% of TEC are shown for reasons of comparison.

Fig 8: Water uptake kinetics of thin (AS/ethylcellulose: 1:1):BMD 4:1 films plasticized with 25 and 30% of TEC (the percentages are indicated in the diagram and refer to the ethylcellulose/AS mass) upon exposure to phosphate buffer pH 6.8, in simulated intestinal fluid during 8 hours. Films consisting only of ethylcellulose: BMD 4:1 plasticized with 25 or 30% of TEC are shown for reasons of comparison.

Fig 9: Dry mass loss kinetics of thin (AS/ethylcellulose: 1:1):BMD 4:1 films plasticized with 25 and 30% of TEC (the percentages are indicated in the diagram and refer to the ethylcellulose/AS mass) upon exposure to phosphate buffer pH 6.8, in simulated intestinal fluid during 8 hours. Films consisting only of ethylcellulose: BMD 4:1 plasticized with 25 or 30% of TEC are shown for reasons of comparison.

Fig 10: In vitro release of 5-ASA from pellets uncoated or coated with ethylcellulose:BMD or with (AS/ethylcellulose): BMD 4:1 under conditions simulating the transit through the upper GIT. The coating level was 20%.

## DETAILED DESCRIPTION OF THE INVENTION

In describing and claiming the present invention, the following terminology will be used in accordance with the definitions set out herein.

As used herein, the term "active ingredient", "drug" or "pharmacologically active ingredient" or any other similar term means any chemical or biological material or compound suitable for administration by the methods previously known in the art and/or by the methods taught in the present invention, that induces a desired biological or pharmacological effect, which may include but is not limited to (1) having a prophylactic effect on the organism and preventing an undesired biological effect such as preventing an infection, (2) alleviating a condition caused by a disease, for example, alleviating pain or inflammation caused as a result of disease, and/or (3) either alleviating, reducing, or completely eliminating the disease from the organism. The effect may be local, such as providing for a local anaesthetic effect, or it may be systemic.

As used herein, the expression "Colonic microflora imbalance" also called Dysbiosis or dysbacteriosis is intended to mean, in the present invention, microbial imbalances as in quality and in quantity in the gastrointestinal tract. This phenomenon is reflected by the quality and quantity of the enzymes present in the colon. Particularly, this altered microflora is observed in the colon of patients suffering from inflammatory bowel diseases, such as Crohn's Disease (CD) and Ulcerative Colitis (UC).

As used herein, the terms "controlled release delivery" or "controlled release" mean that the release of the active ingredient out of the dosage form is controlled with respect to time or with respect to the site of delivery.

As used herein, the expression "a colon targeted delivery" means that the release of the active ingredient out of the dosage form is controlled with respect to time or with respect to the site of delivery specifically in the colon.

The expression "modified starch" should be understood broadly, this expression refers for instance to reticulated or acetylated or hydroxypropylated, or more generally to esterification or etherification starch.

The expression "acetylated starch" or "starch acetate" means a starch modified by acetylation.

Starch acetate has been known to be desirable and biodegradable reactants for the development of food products, fibers, filaments, plastics and other products.

Acetylated starches may have either a low ( <= 1) or high (.about. 2-3) degree of substitution (DS).

DS is determined by the number of free hydroxyls on the amylose and amylopectin units of the starch. Preferably DS is determined by the method recommended by the ISO standard NF EN ISO11213.

Various methods of making starch acetate include treating granular starch with acetic acid or acetic anhydride, either alone or in the presence of a catalyst, such as acetic acid, pyridine, sulfuric acid, or an aqueous alkaline solution.

For low DS starch acetate polymers, this method is usually employed at high pH 7-11 and at room temperature.

High DS starch acetates are prepared similarly, but with longer reaction times. See, e.g., Kirk-Othmer, Encyclopedia of Chemical Technology, 3rd edit, Vol. 21, (John Wiley and Sons, New York, 1978) pp. 504-505; and Food Chemistry, 2d edit., Owen R. Fennema, ed., (Marcel Dekker, Inc., New York, 1985) pp. 118-120.

Acetylation of starch is highly documented See, e.g US 3 795 670, EP 603 837, US 5 667 803, WO 97/03120, WO 98/29455, WO 98/98/29456 et US 2008/0146792.

The determination of molecular mass is performed using the technique of size exclusion chromatography followed by differential refractometry detection. The calibration is performed using polystyrene standards.

The HPSEC chromatography is performed in the following conditions: columns PLgel MIXED-B et PLge150A-Polymer Laboratories; Injected volume: 100 $\mu$l; Flow rate: 0,8 ml/min; columns temperature: 80°C ; Elution solvent : DMAc + 0.05M de NaNO3 ; Analysis time: 60 min.

The term "coat" is used herein to encompass coatings for solid supports and also capsules enclosing fluids and/or solids and the term "coated" is used similarly.

The expression "water insoluble polymer" should be understood broadly, this expression refers to polymers that do not completely dissolve in water, such as for example ethyl cellulose, certain starch derivatives typically acetatylated starches or acrylic acid/methacrylic acid derivatives.

The term "indigestible polysaccharides" as used in the present invention refers to saccharides which are not or only partially digested in the intestine by the action of acids or digestive enzymes present in the human upper digestive tract (small intestine and stomach) but which are at least partially fermented by the human intestinal flora. Indigestible water-soluble polysaccharides that may be employed in preferred embodiments of the invention are xylooligosaccharides, inulin, oligofructoses, fructo-oligosaccharides (FOS), lactulose, galactomannan and suitable hydrolysates thereof, indigestible polydextrose, indigestible dextrins and partial hydrolysates thereof, trans-galacto-oligosaccharides (GOS), xylo-oligosaccharides (XOS), acemannans, lentinans or beta-glucans and partial hydrolysates thereof, polysaccharides-K (PSK), and indigestible maltodextrins and partial hydrolysates thereof.

Polysaccharide-K is also known as polysaccharide-Krestin (PSK) in Japan, and as polysaccharide-peptide (PS-P) in China. Both have the same chemical and structural characteristics. PSK is a proteoglycan found in the polypore fungus *Trametes versicolor* and contains approximately 35% carbohydrate (91% beta-glucan), 35% protein and the remainders are free residues such as sugars, amino acids and moisture. PSK is a mixture of polysaccharides covalently linked to various peptides with an average molecular weight of 100 kilodaltons. The polysaccharide component is in a class of beta-glucans which comprise of glucopyranose units. Structural analysis showed that PSK has a 1, 4-glucan configuration as the main glucoside portion with branches at positions 3 and 6 at a frequency of one branch per several residual groups of 1 - 4 bonds.

As used herein, the term "cereal" is intended to mean, in the present invention, any plant belonging to the Gramineae, preferably wheat, rice, rye, oats, barley, corn, sorghum and millets.

The term "legume" is intended to mean, in the present invention, any plant belonging to the Caesalpinaceae, Mimosaceae or Papilionaceae families and in particular any plant belonging to the Papilionaceae family, such as, for example, pea, bean, broad bean, horse bean, lentil, alfalfa, clover or lupin.

The expression "starch derivative" means a starch that has been enzymatically or chemically treated.

The "coating level" means the difference in weight between uncoated and coated cores that is the weight gain in percentage.

This definition includes in particular all the plants described in any one of the tables present in the paper by R. Hoover et al. entitled "Composition, Structure, Functionality and Chemical Modification of Legume Starches: A Review".

The term "pea" in this instance is considered in its broadest sense and includes in particular:

- ■ all the wild varieties of smooth pea and
- ■ all the mutant varieties of smooth pea and of wrinkled pea, this being the case whatever the uses for which said varieties are generally intended (food for man, animal nutrition and/or other uses).

Said mutant varieties are in particular those referred to as "mutants r", "mutants rb", "mutants rug 3'', mutants rug 4", "mutants rug 5'' and "mutants lam" as described in the paper by C-L Heydley et al. entitled "Developing Novel Pea Starches", Proceedings of the Symposium of the Industrial Biochemistry and Biotechnology Group of the Biochemical Society, 1996, pp. 77-87.

The term "legume starch" is understood to mean any composition extracted, this being the case in whatever way, from a legume as defined hereinabove and having a starch content of greater than 40%, preferably of greater than 50% and more preferably still of greater than 75%, these percentages being expressed in dry weight with respect to the dry weight of said composition.

Furthermore, it is possible to use starches naturally exhibiting an amylose content within the range selected according to the invention. In particular, the starch resulting from legumes may be suitable. In accordance with the present invention, this legume starch exhibits an amylose content of less than 45%, more specifically of between 20 20 and 45%, preferably of between 25 and 44%, and more preferably still of between 32 and 40%.

For the purpose of the invention, the term "ingestible maltodextrin" means maltodextrin containing indigestible glucosidic linkages conferring on those maltodextrins additional properties identical to dietetic fibers such as "branched maltodextrins". As used herein, the term "branched maltodextrins" is intended to mean the ingestible maltodextrins described in patent EP 1 006 128, of which the applicant company is the proprietor.

The branched maltodextrins have a total fiber content of greater than or equal to 50% on a dry basis, determined according to AOAC method No. 2001-03 (2001).

The invention provides novel polymeric film coatings for colon targeting which are adapted to the disease state of the patients suffering from inflammatory bowel diseases.

Novel polymeric films according to the invention serve as substrates for colonic bacteria for healthy patients as for patients suffering from inflammatory bowel diseases and are likely to exhibit beneficial effects on the ecosystem of the GIT of the patients. The polymeric film is specially adapted to the conditions at the target site, also in the disease state and able to deliver pharmacologically active ingredients specifically to the colon.

In the following, the invention will be illustrated by means of the following examples as well as the figures.

Example 1 :

A. Materials and Methods

A.1. Materials

MS 6 HP-PG (a hydroxypropylated and pregelatinized high amylose maize starch (60% amylose) DS:0.05 (EURYLON® 6 HP-PG Roquette Freres, Lestrem, France); MS 6 A-PG (a acetylated and pregelatinized high amylose maize starch (60% amylose) DS:0.05 (EURYLON® 6 A-PG Roquette Freres, Lestrem, France) MS 7 A-PG (a acetylated and pregelatinized high amylose maize starch) (70% amylose) DS:0.05 (EURYLON® 7 A-PG Roquette Freres, Lestrem, France); and an Acetylated potato starch DS:2,7 (AS) 20% amylose; aqueous ethylcellulose dispersion (Aquacoat® ECD 30; FMC Biopolymer, Philadelphia, USA); triethylcitrate (TEC; Morflex, Greensboro, USA); Columbia blood agar, extracts from beef and yeast as well as tryptone (= pancreatic digest of casein) (Becton Dickinson, Sparks, USA); L-cysteine hydrochloride hydrate (Acros Organics, Geel, Belgium); McConkey agar (BioMerieux, Balme-les-Grottes, France); cysteinated Ringer solution (Merck, Darmstadt, Germany).

A.2. Film preparation

No films were obtained by the dispersions MS 6 A-PG, MS 7 A-PG or MS 6 HP-PG dispersions. However, thin, free films were prepared by casting blends of MS 6 HP-PG and aqueous ethylcellulose (plasticized with 25 % TEC); MS 6 A-PG and aqueous ethylcellulose (plasticized with 25 % TEC); MS 7 A-PG and aqueous ethylcellulose (plasticized with 25 % TEC). MS 6 HP-PG MS 6 A-PG or MS 7 A-PG was dispersed in purified water at 65-75° C (5 % w/w). Aqueous AS dispersion (15 % w/w solids content) and aqueous ethylcellulose dispersion (30 % w/w solids content) were plasticized for 24 h with 25 % TEC (w/w, referred to the solids content of the dispersion). The MS 6 HP-PG: ethylcellulose and MS 6 A-PG: ethylcellulose, MS 7 A-PG: ethylcellulose dispersion were blended at room temperature at the following ratios: 1:3 and 1:4 (polymer:polymer, w:w). The mixtures were stirred for 6 h prior to casting.

A.3. Film characterization

The thickness of the films was measured using a thickness gauge (Minitest 600; Erichsen, Hemer, Germany). The mean thickness of all films was in the range of 300-340 $\mu$m. The water uptake and dry mass loss kinetics were measured gravimetrically upon exposure to:

(i) simulated gastric fluid (0.1 M HCl)
(ii) simulated intestinal fluid [phosphate buffer pH 6.8 (USP 30)]
(iii) culture medium inoculated with feces from inflammatory bowel disease patients

Culture medium was prepared by dissolving 1.5 g beef extract, 3 g yeast extract, 5 g tryptone, 2.5 g NaCl and 0.3 g L-cysteine hydrochloride hydrate in 1 L distilled water (pH 7.0 $\pm$ 0.2) and subsequent sterilization in an autoclave. Feces of ulcerative colitis patients were diluted 1:200 with cysteinated Ringer solution; 2.5 mL of this suspension was diluted with culture medium to 100 mL. Film pieces of 1.5 5 cm were placed into 120 mL glass containers filled with 100 mL pre-heated medium, followed by horizontal shaking at 37 °C (GFL 3033, Gesellschaft fuer Labortechnik, Burgwedel, Germany). The incubation with fecal samples was performed under anaerobic conditions (5 % CO2, 10 % H2, 85 % N2). At predetermined time points samples were withdrawn, excess water removed, the films accurately weighed (wet mass) and dried to constant weight at 60 °C (dry mass). The dry film mass (%) at time t was calculated as follows:

$$dry\ film\ mass\ (\%)\ (t) = \frac{dry\ mass\ (t)}{dry\ mass\ (t\ =\ 0)} \cdot 100\ \%$$

B. Results and discussion Film properties in the upper GIT

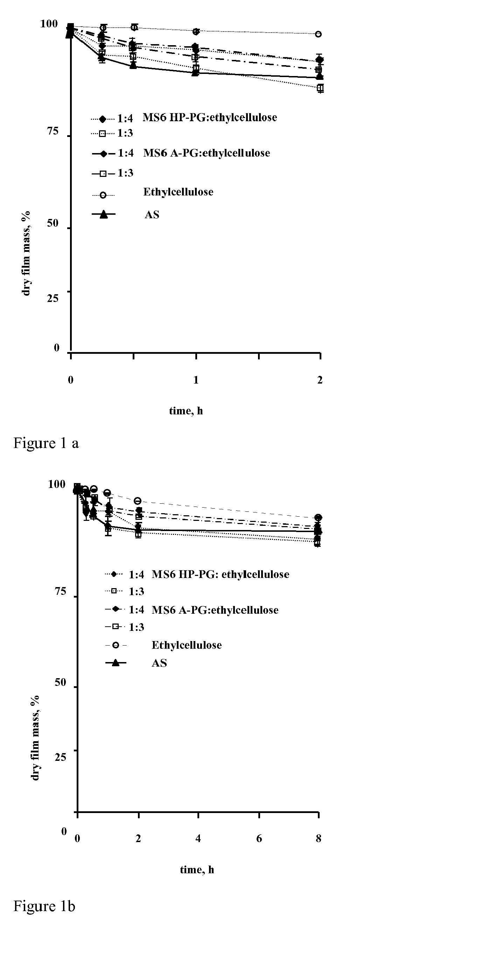

The permeability of a polymeric system for a drug strongly depends on its water content and dry mass, which determines the density and mobility of the macromolecules.. For instance, in dry hydroxypropyl methylcellulose (HPMC)-based matrix tablets the apparent diffusion coefficient of a drug approaches zero, whereas in a completely hydrated HPMC gel diffusivities can be reached, which are in the same order of magnitude as in aqueous solutions. With increasing water content the macromolecular mobility significantly increases and, thus, the free volume available for diffusion. In some systems, the polymer undergoes a glassy-to-rubbery phase transition as soon as a critical water content is reached. This leads to a significant, stepwise increase in polymer and drug mobility. Thus, the water content of a polymeric film coating can give important insight into the macromolecular mobility and, hence, permeability for a drug. The ideal film looses only minor amounts of dry mass at a low rate (or no mass at all), assuring dense polymeric networks which are poorly permeable for the incorporated drug under these conditions. The dry mass loss behavior of thin polymeric films serves as an indicator for the coatings' permeability for the drug, and, hence, potential to suppress premature release within the upper GIT. If the films loose significant amounts of dry mass upon exposure to the release media, the coatings can be expected to become permeable for many drugs, in particular those with a low molecular weight such as 5-aminosalicylic acid (5-ASA, 153.1 Da). Figures 1a and 1b illustrate the experimentally determined dry mass loss of thin films consisting of various types of modified starches and ethyl cellulose blends in 0.1 N HCl and phosphate buffer pH 6.8, respectively. In the films containing high amylose starches (MS 6 A-PG, MS6 HP-PG and MS 7 A-PG) tThe presence of ethyl cellulose in all films allows the formations of thin films the films limits the dry mass loss. By the opposite AS mix give well formed thin films without adding ethyl cellulose. Thus, this last acetylated starch may be used alone in upper GIT resistant films avoiding premature dissolution. ThusAlso, AS limits the dry mass loss (Figure 1), so it maymay be a good candidate for replacing ethyl cellulose in upper GIT resistant films.

Example 2 :

A. Materials and Methods

A.1. Materials

MS6 HP-PG (a hydroxypropylated and pregelatinized high amylose maize starch (60% amylose) DS:0.05 (EURYLON® 6 HP-PG Roquette Freres, Lestrem, France); Acetylated potato starch DS:2,7 20% amylose (AS) and Branched maltodextrin (BMD) [a branched maltodextrin with non digestible glycoside linkages: $\alpha$-1,2 and $\alpha$-1,3, NUTRI-OSE® FB 06 Roquette Frères] ; aqueous ethylcellulose dispersion (Aquacoat® ECD 30; FMC Biopolymer, Philadelphia, USA); triethylcitrate (TEC; Morflex, Greensboro, USA); Columbia blood agar, extracts from beef and yeast as well as tryptone (= pancreatic digest of casein) (Becton Dickinson, Sparks, USA); L-cysteine hydrochloride hydrate (Acros Organics, Geel, Belgium); McConkey agar (BioMerieux, Balme-les-Grottes, France); cysteinated Ringer solution (Merck, Darmstadt, Germany).

A.2. Film preparation

Thin, free films were prepared by casting blends of Eurylon 6 HP-PG and aqueous AS (plasticized with 25 % TEC), or BMD and aqueous ethylcellulose dispersion (plasticized with 25 % TEC into Teflon moulds and subsequent controlled drying (1 d at 60° C). Eurylon 6 HP-PG was dispersed in purified water at 65-75° C (5 % w/w). BMD was dissolved in purified water (5 % w/w). Aqueous AS dispersion (15 % w/w solids content) and aqueous ethylcellulose dispersion (30 % w/w solids content) were plasticized for 24 h with 25 % TEC (w/w, referred to the solids content of the dispersion). The Eurylon 6 HP-PG and AS dispersion as well as BMD:aqueous ethylcellulose dispersion were blended at room temperature at the following ratios: 0:1, 1:2, 1:3, 1:4 and 1:5 (polymer:polymer, w:w). The mixtures were stirred for 6 h prior to casting.

A.3. Film characterization

The thickness of the films was measured using a thickness gauge (Minitest 600; Erichsen, Hemer, Germany). The mean thickness of all films was in the range of 300-340 $\mu$m. The water uptake and dry mass loss kinetics were measured gravimetrically upon exposure to:

    (i) simulated gastric fluid (0.1 M HCl)
    (ii) simulated intestinal fluid [phosphate buffer pH 6.8 (USP 30)]
    (iii) culture medium inoculated with feces from inflammatory bowel disease patients

Culture medium was prepared by dissolving 1.5 g beef extract, 3 g yeast extract, 5 g tryptone, 2.5 g NaCl and 0.3 g L-cysteine hydrochloride hydrate in 1 L distilled water (pH 7.0 $\pm$ 0.2) and subsequent sterilization in an autoclave. Feces of ulcerative colitis patients were diluted 1:200 with cysteinated Ringer solution; 2.5 mL of this suspension was diluted with culture medium to 100 mL. Film pieces of 1.5 x 5 cm were placed into 120 mL glass containers filled with 100 mL pre-heated medium, followed by horizontal shaking at 37 °C (GFL 3033, Gesellschaft fuer Labortechnik, Burgwedel, Germany). The incubation with fecal samples was performed under anaerobic conditions (5 % $CO_2$, 10 % $H_2$, 85 % $N_2$). At predetermined time points samples were withdrawn, excess water removed, the films accurately weighed (wet mass) and dried to constant weight at 60 °C (dry mass). The water content (%) and dry film mass (%) at time t were calculated as follows:

$$water\ content\ (\%)\ (t) = \frac{wet\ mass\ (t) - dry\ mass\ (t)}{wet\ mass\ (t)} \cdot 100\ \%\ (1)$$

$$dry\ film\ mass\ (\%)\ (t) = \frac{dry\ mass\ (t)}{dry\ mass\ (t = 0)} \cdot 100\ \%\ \ \ (2)$$

B. Results and discussion

*B.1. MS6 HP-PG:AS comparing* to *MS6 HP-PG:ethylcellulose blends*

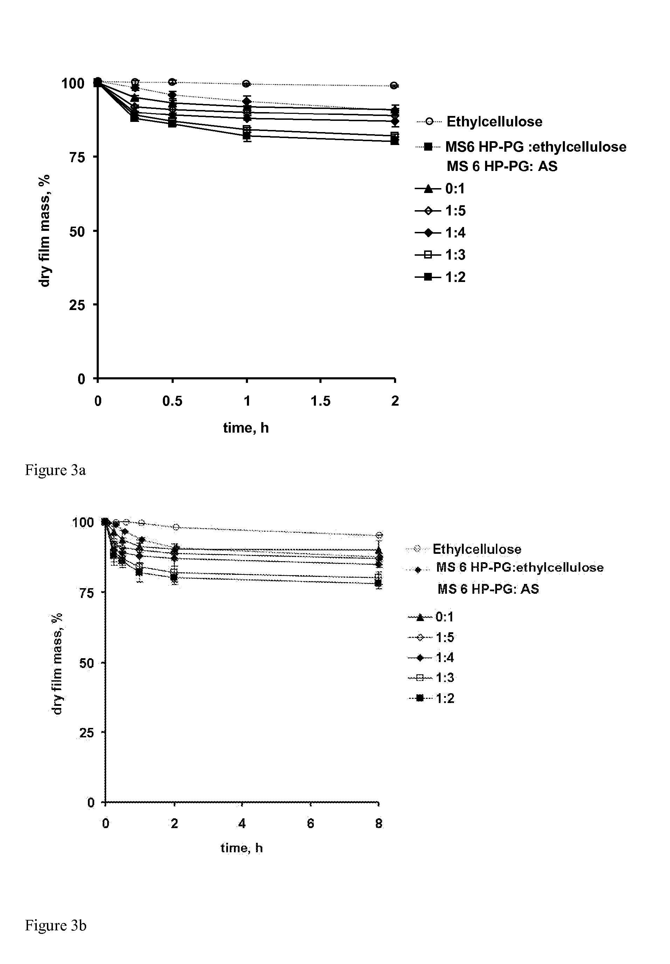

MS6 HP- PG is a hydroxypropylated and pregelatinized high amylose maize starch (60% amylose) (EURYLON® 6 HP-PG Roquette Freres, Lestrem, France). The dry mass loss of thin films consisting of MS6 HP-PG: AS blends was more pronounced than that of the MS6 HP-PG: ethylcellulose upon exposure to 0.1 M HCl and phosphate buffer pH 6.8 (Figure 3). However, the dry mass loss is sufficiently low to confirm the possible use of AS as a potential substitute of ethylcellulose in colon targeting. Similar rates and extents of the dry mass loss were observed for all of the investigated polymer blend ratios. Thus, as for MS6 HP- PG: ethylcellulose and MS6 HP-PG: AS blends elevated coating levels are likely to be required to suppress premature release of freely water-soluble, low molecular weight drugs in the upper GIT.
The key properties of thin polymeric films consisting of indigestible polysaccharide: water insoluble polymer blends exhibiting an interesting potential to provide site specific drug delivery to the colon (and being adapted to the pathophysiology of inflammatory bowel disease patients) can effectively be adjusted by varying the polymer blend ratio and type of polysaccharide. This includes the water uptake and dry mass loss kinetics as well as the mechanical properties of the films before and upon exposure to aqueous media simulating the contents of the upper GIT. Thus, broad ranges of film coating properties can easily be provided, being adapted to the needs of the respective drug treatment (e.g., osmotic activity of the core formulation and administered dose)

B.2. Film properties in the colon

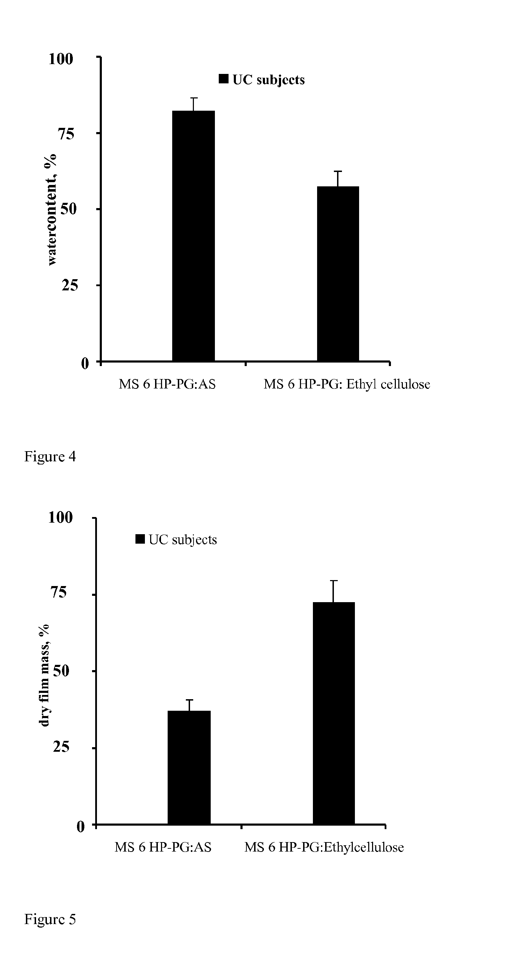

Once the colon is reached, the polymeric film coatings should become permeable for the drug. This can for instance be induced by (partial) enzymatic degradation. Importantly, the concentrations of certain enzymes are much higher in the colon than in the upper GIT. This includes enzymes, which are produced by the natural microflora of the colon (this part of the GIT contains much more bacteria than the stomach and small intestine). However, great caution must be paid when using this type of colon targeting approach, because the microflora of patients suffering from inflammatory bowel diseases can be significantly different from the microflora of healthy subjects. Thus, the drug delivery system must be adapted to the disease state of the patient. Table 1 shows for instance the concentrations of the bacteria determined in the fecal samples of the healthy subjects as well as of the Crohn's Disease and Ulcerative Colitis patients included in this study. Importantly, there were significant differences, in particular with respect to the concentrations of Bifidobacterium (being able to degrade complex polysaccharides due to multiple extracellular glycosidases) and Escherichia coli, which where present at much higher concentrations in the feces of healthy subjects compared to the feces of the inflammatory bowel disease patients. In contrast, the fecal samples of the Crohn's Disease and Ulcerative Colitis patients contained lactose negative E. coli, Citrobacter freundii, Klebsiella pneumoniae, Klebsiella oxytoca and Enterobacter cloacae, which were not detected in healthy subjects. Thus, there are fundamental differences in the quality and quantity of the microflora, which must be taken into account: Polymeric film coatings, which allow for colon targeting under physiological conditions in a healthy volunteer, might fail under the pathophysiological conditions in the disease state of a patient. To address this very crucial point, which is very often neglected, the water uptake and dry mass loss of thin films consisting of MS6 HP-PG:AS blend were determined upon exposure to fecal samples from Crohn's Disease and Ulcerative Colitis patients. This resulted were further compared with MS6 HP-PG:ethyl cellulose blends analysis (Figures 4 and 5). Appropriate films should take up considerable amounts of water and show significant dry mass loss upon exposure to patients' feces in order to induce drug release at the site of inflammation in the colon. As it can be seen in Figures 4 and 5, films containing AS are very promising.

The novel polymeric film coatings identified for colon targeting comprises a water insoluble polymer that is AS and an indigestible polysaccharide. These novel polymeric film coatings are likewise adapted to the disease state of the patients.

Example 3 :

A. Materials and Methods

A.1. Materials

Branched Maltodextrin (BMD) (a water-soluble, branched dextrin with high fiber contents obtained from wheat starch; (Nutriose® FB 06 Roquette Freres, Lestrem, France); Acetylated potato starch DS:2,7 (AS) 20% of amylose content (AS)(an acetylated starch) (Roquette Freres, Lestrem, France); aqueous ethylcellulose dispersion (Aquacoat® ECD 30; FMC Biopolymer, Philadelphia, USA); triethylcitrate (TEC; Morflex, Greensboro, USA). 5-aminosalicylic acid (5-ASA; Sigma-Aldrich, Isle d'Abeau Chesnes, France); microcrystalline cellulose (Avicel PH 101; FMC Biopolymer, Brussels, Belgium); bentonite and polyvinylpyrrolidone (PVP, Povidone K 30) (Cooperation Pharmaceutique Francaise, Melun, France)

A.2. Preparation of thin, polymeric films

Thin, free films were prepared by casting blends of BMD, aqueous AS dispersion (15 % solid content) and ethylcellulose dispersion (plasticized with 25 % or 30 % TEC) into Teflon moulds and subsequent controlled drying (1 d at 60° C). BMD was dissolved in purified water (5 % w/w). Aqueous AS dispersion (15 % w/w solids content) was blended with an aqueous dispersion of ethylcellulose at the ratio 1:1 and plasticized for 24 h with 25 % or 30 % TEC (w/w, referred to the solids' content of the dispersion). The BMD and AS/ethylcellulose solution/dispersion were blended at room temperature at the ratio 1:4 (w:w). The mixtures were stirred for 6 h prior to casting. For reasons of comparison, the BMD was blended only with plasticized (25 % or 30 % TEC) aqueous ethylcellulose dispersion.

A.3. Film characterization

The thickness of the films was measured using a thickness gauge (Minitest 600; Erichsen, Hemer, Germany). The mean thickness of all films was in the range of 300-340 $\mu$m. The water uptake and dry mass loss kinetics were measured gravimetrically upon exposure to:

(i) simulated gastric fluid (0.1 M HCl)
(ii) simulated intestinal fluid [phosphate buffer pH 6.8 (USP 30)]

At predetermined time points samples were withdrawn, excess water removed, the films accurately weighed (wet mass) and dried to constant weight at 60°C (dry mass). The water content (%) and dry film mass (%) at time t were calculated as follows:

$$Water\ content\ (\%)\ (t) = \frac{wet\ mass\ (t) - dry\ mass\ (t)}{wet\ mass\ (t)} \cdot 100\ \%\ (1)$$

$$Dry\ film\ mass\ (\%)\ (t) = \frac{dry\ mass\ (t)}{dry\ mass\ (t = 0)} \cdot 100\ \%\ \ (2)$$

A.4. Preparation of drug-loaded pellet cores

Drug-loaded pellet cores (diameter: 710-1000 μm; 60 % 5-ASA, 32 % microcrystalline cellulose, 4 % bentonite, 4 % PVP) were prepared by extrusion and spheronization. The powders were blended in a high speed granulator (Gral 10; Collette, Antwerp, Belgium) and purified water was added until a homogeneous mass was obtained. The wetted powder mixture was passed through a cylinder extruder (SK M/R; Alexanderwerk, Remscheid, Germany). The extrudates were subsequently spheronized at 520 rpm (Spheronizer Model 15; Calveva, Dorset, UK) and dried in a fluidized bed (ST 15; Aeromatic, Muttenz, Switzerland) at 40°C for 30 min.

A.5. Preparation of coated pellets

BMD was dissolved in purified water (5 % w/w). Aqueous AS dispersion (15 % w/w solids content) was blended with an aqueous dispersion of ethylcellulose at the ratio 1:1 and plasticized for 24 h with 30 % (w/w, referred to the solids' content of the dispersion). The BDM and AS/ethylcellulose solution/dispersion were blended at room temperature at the ratio 1:4 (w:w). The mixtures were stirred for 6 h prior to coating. For reasons of comparison, BMD was blended with plasticized ethylcellulose (25 % TEC) at room temperature at ratio of 1:4 (w/w). The drug-loaded pellet cores were coated in a fluidized bed coater equipped with a Wurster insert (Strea 1; Aeromatic-Fielder, Bubendorf, Switzerland) until a weight gain of 20 % (w/w) was achieved. The process parameters were as follows: inlet temperature = 39 $\pm$ 2 °C, product temperature = 40 $\pm$ 2 °C, spray rate = 1.5-3 g/min, atomization pressure = 1.2 bar, nozzle diameter = 1.2 mm. After coating, the beads were further fluidized for 10 min and subsequently cured in an oven for 24 h at 60 °C.

A.6. In vitro drug release

Pellets were placed into 120 mL plastic containers, filled with 100 mL dissolution medium: 0.1 M HCl during the first 2 h, then complete medium change to phosphate buffer pH 6.8 (USP 30). The flasks were agitated in a horizontal shaker (80 rpm; GFL 3033; Gesellschaft fuer Labortechnik, Burgwedel, Germany). At pre-determined time points, 3 mL samples were withdrawn and analyzed UV-spectrophotometrically ($\lambda = 302.6$ nm in 0.1 M HCl; $\lambda = 330.6$ nm in phosphate buffer pH 6.8) (Shimadzu UV-1650, Champs sur Marne, France). Each experiment was conducted in triplicate.

B. Results and discussion

B.1. Film properties in the upper GIT

It is well known that the plasticizer content can significantly affect the mechanical properties of polymeric films. In order to evaluate the importance of this phenomenon for the investigated coating mix containing a reduced content of ethyl cellulose that is (AS/ethylcellulose):BMD blends, the percentage of incorporated TEC was increased from 25 to 30% w/w (referred to the ethylcellulose content). Effect of TEC in ethyl cellulose films has been well investigated. However, this effect may vary in starch/ethyl cellulose films. Indeed, in ethyl cellulose films, TEC contents below 25% w/w would render the fusion of the ethylcellulose nanoparticles during film formation difficult, the mobility of the polymer chains being crucial for this step. TEC contents higher than 30% w/w significantly increase the sticking tendency during coating and curing and should, thus, be avoided

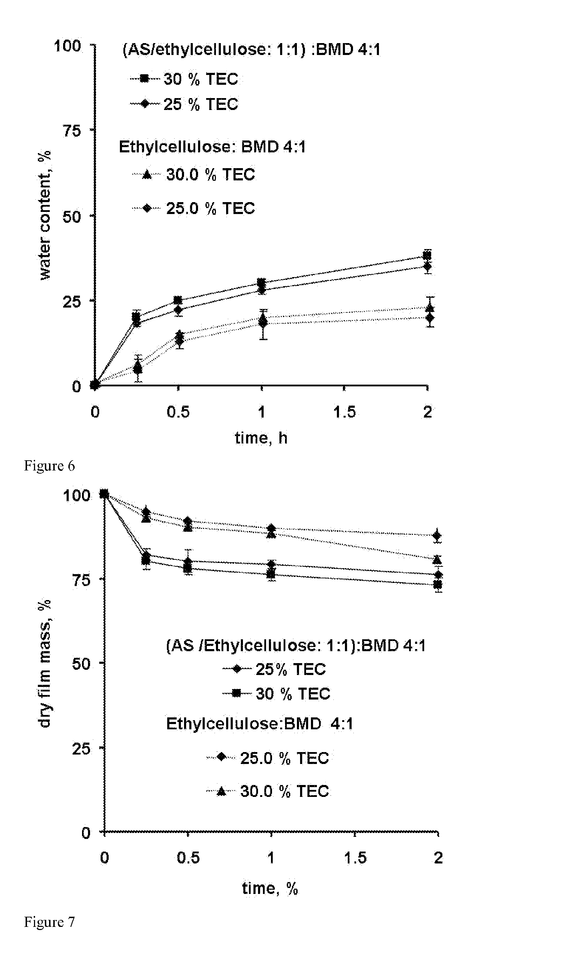

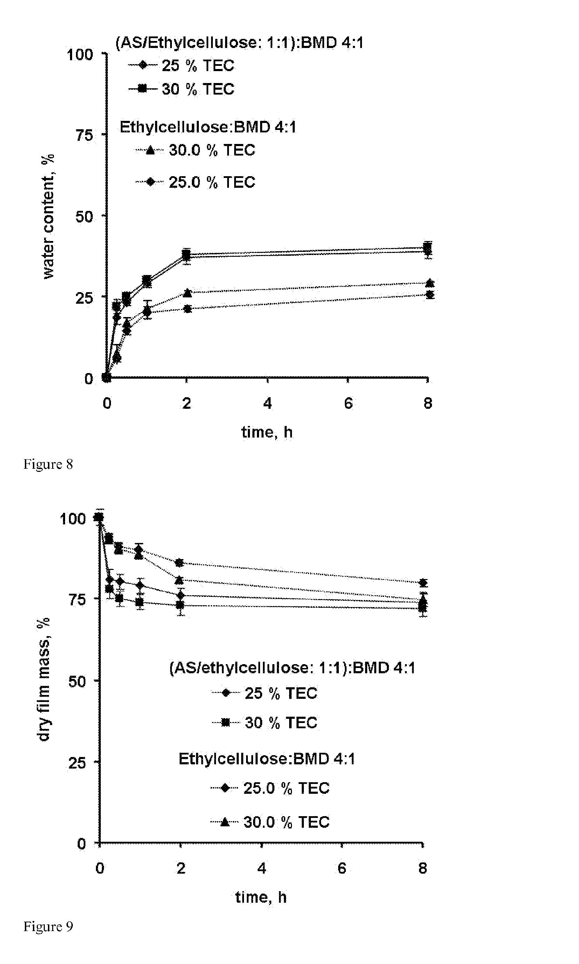

However, when increasing the percentage of the water-soluble plasticizer TEC in the polymeric films, also the rates and extents of the systems' water uptake and dry mass loss upon exposure to aqueous media can be expected to increase. This might potentially lead to significantly increased drug permeability of the polymeric films, resulting in potential premature drug release within the upper GIT. To estimate the importance of these phenomena, the water uptake and dry mass loss kinetics of the investigated films were monitored upon exposure to 0.1 N HCl for 2 h (figures 6 and 7) and upon exposure to phosphate buffer pH 6.8 for 8 h (figures 8 and 9). Importantly, the resulting changes in the water uptake and dry mass loss kinetics were only minor when increasing the initial TEC content from 25 to 30 %, irrespective of the water insoluble polymers mix. Thus, the mechanical stability of (AS/ethyl cellulose):BMD films can efficiently be improved by increasing the plasticizer level, without loosing the systems' capability to limit drug release within the upper GIT.

*B.2. Drug release in the upper gastro intestinal tract*

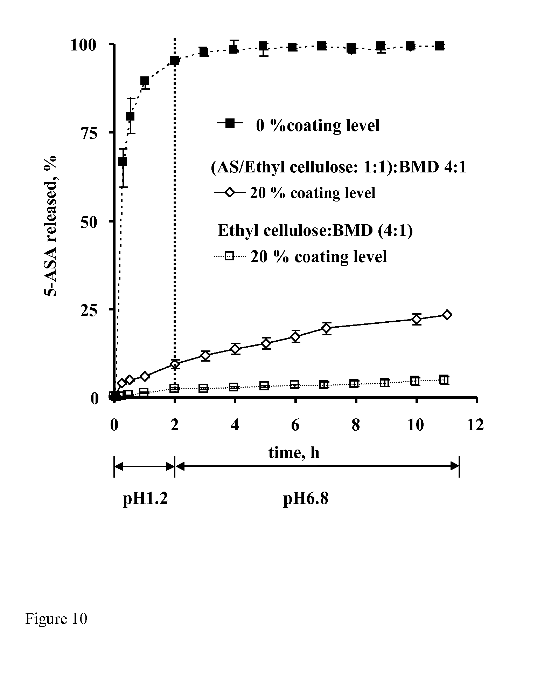

Ideally, no or very little drug should be released from the dosage form in the stomach and small intestine. The curves in Figure 10 show the experimentally determined drug release kinetics from pellets coated with 20% (AS/ethyl cellulose):BMD with AS:ethyl cellulose ratio at 1:1 and the water insoluble polymers composition : indigestible polysaccharide ratio is 4:1((1:1)4:1) in comparison with ethylcellulose:BMD blend with a ratio (4:1) at a coating level of 0 and 20 % (w/w) into: 0.1 M HCl (for 2 h), followed by phosphate buffer pH 6.8 (for 9 h) at 37 °C. As it can be seen, 5-amino salicylic acid was rapidly released from uncoated pellets. The film containing AS/ethylcellulose mixes are able to slow down drug release. However, comparing with the ethyl cellulose films, the films obtained by a partial ethyl cellulose replacement exhibit a relative permeability to 5-ASA. It must be noted that this active principle is a small molecule.

Table 1:

| | Healthy subjects | Crohn's Disease | Ulcerative Colitis |
|---|---|---|---|
| Number | 10 | 11 | 5 |
| Mean age | 40 +/-15 | 32+/-12 | 36+/-20 |
| Mean total counts [log UFC/g] | 9.88+/-0,48 | 9.15+/-1.30 | 9.88+/-0.57 |
| | | | |
| Number of strains | 28 | 34 | 14 |
| Mean | 2.8 | 3.1 | 2.8 |
| | | | |
| **Anaerobes** | | | |
| *Bacteroides* | 9 | 10 | 3 |
| *Prevotella* | 2 | 2 | 2 |
| *Fusobacterium* | 3 | 3 | 2 |
| *Veillonella* | 0 | 0 | 1 |
| *Clostridium* | 0 | 5 | 1 |
| *Bifidobacterium* | 9 | 3 | 1 |
| Other Gram + rods | 3 | 2 | 2 |
| Gram + cocci | 1 | 2 | 0 |
| | | | |
| **Aerobes** | | | |
| Enterobacteria | 1 | 3 | 2 |
| *Escherichia coli* | 1 | 2 | 1 |
| *Citrobacter freundii* | 0 | 2 | 1 |
| *Lactobacillus* | 0 | 2 | 0 |
| *Streptococcus* | 0 | 2 | 0 |
| | | | |
| | | | |
| Mean counts McConkey agar | 6.30+/-1.19 | 7.16+/-1.48 | 8.01+/-1.06 |
| Number of strains | 10 | 14 | 8 |
| | | | |
| *Escherichia coli* | 10 | 6 | 4 |
| *E. coli* lac- | 0 | 1 | 0 |
| *Citrobacter freundii* | 0 | 3 | 1 |
| *Klebsiella pneumoniae* | 0 | 1 | 1 |
| *Klebsiella oxytoca* | 0 | 2 | 0 |
| *Enterobacter cloacae* | 0 | 1 | 0 |
| Other Gram - rods | 0 | 0 | 1 |

**Claims**

1. A colon targeted delivery dosage form for controlled release of an active ingredient, comprising an active ingredient coated with a polymeric mixture of:

   o a water insoluble polymer composition containing at least a starch acetate, and
   o an indigestible polysaccharide composition.

2. The colon targeted delivery dosage form according to claim 1, wherein the starch acetate has an amylose content of less than 55%, this percentage being expressed in dry weight with respect to the dry weight of starch present in said composition.

3. The colon targeted delivery dosage form according to claim 1, wherein the water insoluble polymer composition further contains another water insoluble polymer.

4. The colon targeted delivery dosage form according to claim 3, wherein the water insoluble polymer is selected from the group consisting of ethyl cellulose, cellulose derivatives, acrylic and/or methacrylic ester polymers, polymers or copolymers of acrylate or methacrylate polyvinyl esters, starch derivatives, polyvinyl acetates, polyacrylic acid esters, butadiene styrene copolymers methacrylate ester copolymers, cellulose acetate phtalate, polyvinyl acetate phtalate, shellac, methacrylic acid copolymers, cellulose acetate trimellitate, hydroxypropyl methylcellulose phthalate, zein.

5. The colon targeted delivery dosage form according to claims 3 or 4, wherein the ratio between the starch acetate and the other water insoluble polymers is between 1:2 and 1:8.

6. The colon targeted delivery dosage form according to any one of claims 1 to 5, wherein the starch acetate has a degree of substitution of 1.6 to 3.

7. The colon targeted delivery dosage form according to any one of claims 1 to 6, wherein the indigestible polysaccharide composition comprises at least one indigestible polysaccharide selected from the group consisting of starch, xylooligosaccharides, inulin, oligofructoses, fructo-oligosacharides (FOS), lactulose, galactomannan and suitable hydrolysates thereof, indigestible polydextrose, indigestible dextrin and partial hydrolysates thereof, trans-galacto-oligosaccharides (GOS), xylo-oligosaccharides (XOS), acemannan, lentinan or beta-glucan and partial hydrolysates thereof, polysaccharides-K (PSK), and indigestible maltodextrin and partial hydrolysates thereof, preferably an indigestible dextrin or an indigestible maltodextrin.

8. The colon targeted delivery dosage form according to claim 7, wherein the indigestible polysaccharide is an indigestible maltodextrin or indigestible dextrin having between 15 and 50% of 1->6 glucoside linkages, a reducing sugar content of less than 20%, a polymolecularity index of less than 5% and a number-average molecular mass $M_n$ at most equal to 4500 g/mol.

9. The colon targeted delivery dosage form according to claim 7, wherein the indigestible polysaccharide is a starch selected from legume or cereal starch.

10. The colon targeted delivery dosage form according to any one of claims 1 to 9, wherein the indigestible polysaccharide composition:water insoluble polymer composition ratio in weight is between 1:2 and 1:8.

11. The colon targeted delivery dosage form according to any one of claims 1 to 10, wherein the polymeric mixture comprises a plasticizer.

12. The colon targeted delivery dosage form according to claim 11, wherein the plasticizer content is between 15% to 50% w/w referred to the water insoluble polymer composition content.

13. The colon targeted delivery dosage form according to any one of claims 1 to 12, wherein the colon targeted delivery dosage form is an oral formulation and has a gastric resistance.

14. The colon targeted delivery dosage form according to any one of claims 1 to 13, wherein the colon targeted delivery dosage form is in a solid form.

**15.** A method for preparing a colon targeted delivery dosage form for controlled release of an active ingredient in the colon of patients having a colonic microflora imbalance or in the colon of healthy subjects, said method comprising:

    o forming a polymeric mixture of:

        • a water insoluble polymer composition containing at least a starch acetate and
        • an indigestible polysaccharide composition

    o coating said active ingredient in the polymeric mixture.

Figure 1 a

Figure 1b

Figure 2a

Figure 2b

Figure 3a

Figure 3b

Figure 4

Figure 5

Figure 6

Figure 7

Figure 8

Figure 9

Figure 10

Europäisches
Patentamt

European
Patent Office

Office européen
des brevets

# EUROPEAN SEARCH REPORT

**Application Number**

EP 10 30 5452

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Karrout, Youness: "Innovative drug delivery systems for colon targeting", Dissertation , 5 December 2008 (2008-12-05), pages 1-132, XP002610371, Retrieved from the Internet: URL:http://www.diss.fu-berlin.de/diss/receive/FUDISS_thesis_000000006476 * page 25, line 1 - page 32, last line; table 3 * * page 35, line 1 - page 45, paragraph 2; figures 4-9 * * page 51, line 1 - page 65, last line; figures 16-23 * * page 66, line 1 - page 104, last line; figures 24-48 * & Karrout, Youness: "Innovative drug delivery system for colon targeting", Dissertation , 5 December 2008 (2008-12-05), pages 1-132, Retrieved from the Internet: URL:http://www.diss.fu-berlin.de/diss/servlets/MCRFileNodeServlet/FUDISS_derivate_000000004788/Elektronische_Ver%C3%B6ffentlichung_Karrout.pdf?hosts= ----- -/-- | 1-15 | INV. A61K9/50 A61K9/20 A61K47/38 A61K47/36 A61K9/28 A61K9/48 |
| | | | TECHNICAL FIELDS SEARCHED (IPC) |
| | | | A61K |

The present search report has been drawn up for all claims

| Place of search | Date of completion of the search | Examiner |
|---|---|---|
| Munich | 19 November 2010 | Toulacis, C |

EPO FORM 1503 03.82 (P04C01)

| | Europäisches Patentamt European Patent Office Office européen des brevets | **EUROPEAN SEARCH REPORT** | Application Number EP 10 30 5452 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NUTAN M T H ET AL: "Optimization and characterization of controlled release multi-particulate beads coated with starch acetate", INTERNATIONAL JOURNAL OF PHARMACEUTICS, ELSEVIER BV, NL, vol. 294, no. 1-2, 27 April 2005 (2005-04-27), pages 89-101, XP004825463, ISSN: 0378-5173, DOI: DOI:10.1016/J.IJPHARM.2005.01.013 * the whole document * ----- | 1-15 | |
| A | TARVAINEN M ET AL: "Aqueous starch acetate dispersion as a novel coating material for controlled release products", JOURNAL OF CONTROLLED RELEASE, ELSEVIER, AMSTERDAM, NL, vol. 96, no. 1, 16 April 2004 (2004-04-16), pages 179-191, XP004500743, ISSN: 0168-3659, DOI: DOI:10.1016/J.JCONREL.2004.01.016 * the whole document * ----- | 1-15 | TECHNICAL FIELDS SEARCHED (IPC) |
| A | WO 97/02018 A1 (ALKO GROUP LTD [FI]; URTTI ARTO OLAVI [FI]; PELTONEN SOILI HELLEVI [FI] 23 January 1997 (1997-01-23) * the whole document * ----- | 1-15 | |

The present search report has been drawn up for all claims

| Place of search | Date of completion of the search | Examiner |
|---|---|---|
| Munich | 19 November 2010 | Toulacis, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 10 30 5452

This annex lists the patent family members relating to the patent documents cited in the above-mentioned European search report.
The members are as contained in the European Patent Office EDP file on
The European Patent Office is in no way liable for these particulars which are merely given for the purpose of information.

19-11-2010

| Patent document cited in search report | Publication date | Patent family member(s) | Publication date |
|---|---|---|---|
| WO 9702018      A1 | 23-01-1997 | AT      219663 T<br>AU      703085 B2<br>AU     5503296 A<br>CA     2222372 A1<br>DE    69622038 D1<br>DE    69622038 T2<br>DK      850049 T3<br>EP     0850049 A1<br>ES     2176452 T3<br>HU     9900304 A2<br>JP    11508581 T<br>PT      850049 E | 15-07-2002<br>18-03-1999<br>05-02-1997<br>23-01-1997<br>01-08-2002<br>13-02-2003<br>21-10-2002<br>01-07-1998<br>01-12-2002<br>28-06-1999<br>27-07-1999<br>31-10-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3795670 A
- EP 603837 A
- US 5667803 A
- WO 9703120 A
- WO 9829455 A
- WO 989829456 A
- US 20080146792 A
- EP 1006128 A

**Non-patent literature cited in the description**

- **KIRK-OTHMER.** Encyclopedia of Chemical Technology. John Wiley and Sons, 1978, vol. 21, 504-505
- Food Chemistry. Marcel Dekker, Inc, 1985, 118-120
- **R. HOOVER et al.** *Composition, Structure, Functionality and Chemical Modification of Legume Starches: A Review*
- **C-L HEYDLEY et al.** Developing Novel Pea Starches. *Proceedings of the Symposium of the Industrial Biochemistry and Biotechnology Group of the Biochemical Society,* 1996, 77-87